# EUROPÄISCHE PATENTSCHRIFT

(11) **EP 3 401 722 B1**
(45) Veröffentlichungstag und Bekanntmachung des Hinweises auf die Patenterteilung: **17.08.2022**
(21) Anmeldenummer: 18159054.8
(22) Anmeldetag: 28.02.2018
(51) Int. Cl.: G02B 23/24, G02B 7/02, A61B 1/00, A61B 1/07

(54) **LINSE FÜR EIN DISTALES ENDE EINES OPTIKKANALS EINES ENDOSKOPSCHAFTES**
LENS FOR A DISTAL END OF AN OPTICAL CHANNEL OF AN ENDOSCOPE SHAFT
LENTILLE POUR UNE EXTRÉMITÉ DISTALE D'UN CANAL OPTIQUE D'UNE TIGE D'ENDOSCOPE

(30) Priorität: 09.05.2017 DE 102017109913
(43) Veröffentlichungstag der Anmeldung: 14.11.2018
(73) Patentinhaber: Henke-Sass, Wolf GmbH, 78532 Tuttlingen (DE)
(72) Erfinder: REHE, Oliver, 78573 Wurmlingen (DE); MATTES, Andreas, 78589 Dürbheim (DE)
(74) Vertreter: Patentanwälte Geyer, Fehners & Partner mbB

(56) Entgegenhaltungen:
- DE-A1- 19 502 006
- JP-A- 2003 230 534
- US-A1- 2014 275 786

## Beschreibung

Die vorliegende Erfindung betrifft eine Linse für ein distales Endes eines Optikkanals eines Endoskopschaftes mit den Merkmalen des Oberbegriffs des Anspruches 1.

Solche Linsen sind z.B. aus der US 2014/275786 A1 bekannt und können im distalen Ende eines Endoskops eingelötet sein, um das distale Ende abzudichten. Dadurch kann auf ein sonst übliches zusätzliches Planglas verzichtet werden, das sonst die distale Abdichtung realisieren würde. Die JP 2003 230534 A zeigt eine Linse für ein distales Ende eines Optikkanals eines Endoskopschafts, wobei die Linsen auf einer Umfangsfläche eine lichtabsorbierende Beschichtung aufweisen. Die DE 195 02 006 A1 zeigt einen Infrarotstrahler mit einem Gehäuse, das eine mittels einem optischen Fenster verschlossene Öffnung aufweist. Das Fenster ist am Rand der Öffnung eingelötet und weist an der Umfangsfläche eine Haftvermittlungsschicht auf, auf die eine lötbare Dünnschicht aufgebracht ist, die sich auch auf einen an die Umfangsfläche anschließenden Abschnitt der Innenseite des Fenster erstreckt. Zwischen der lötbaren Dünnschicht und dem Rand der Öffnung ist eine Weichlotschicht.

Es hat sich jedoch gezeigt, dass bei der Variante der Abdichtung des distalen Endes mit einer Linse die Qualität der Abbildung der gesamten Endoskopoptik den stetig wachsenden Anforderungen nicht mehr vollständig genügt.

Es ist daher Aufgabe der Erfindung, eine verbesserte Linse zur Abdichtung eines distalen Endes eines Optikkanals eines Endoskopschaftes bereitzustellen.

Die Erfindung ist im Anspruch 1 definiert. Vorteilhafte Weiterbildungen sind in den abhängigen Ansprüchen angegeben.

Die erfindungsgemäße Linse für ein distales Endes eines Optikkanals eines Endoskopschaftes weist eine Außenseite, eine Innenseite sowie eine die beiden Seiten verbindende Umfangsfläche auf, wobei auf der Umfangsfläche und einem daran anschließenden Bereich der Innenseite eine lichtabsorbierende Beschichtung aufgebracht ist. Des Weiteren ist im Bereich der Umfangsfläche auf der lichtabsorbierenden Beschichtung eine lötbare Schicht aufgebracht. Damit ist es möglich, die Linse im distalen Ende so einzulöten, dass das distale Ende abgedichtet ist. Da die lichtabsorbierende Beschichtung auf der Umfangsfläche und dem daran anschließenden Bereich der Innenseite ausgebildet ist, findet eine ausgezeichnete Falschlicht- und Streulichtunterdrückung statt, so dass der Kontrast der Abbildung der gesamten Endoskopoptik verbessert und unerwünschte Doppel- und Geisterbilder unterdrückt werden können.

Die erfindungsgemäße Linse ist bevorzugt eine Linse mit negativer Brechkraft. Die Innenseite der Linse kann einen gekrümmten Abschnitt (z.B. konkav gekrümmt) aufweisen. Der gekrümmte Abschnitt, der zur Abbildung benötigt wird, wird natürlich nicht mit der lichtabsorbierenden Beschichtung versehen. Die lichtabsorbierende Beschichtung ist bevorzugt in einem Bereich der Innenseite aufgebracht, der an den gekrümmten Abschnitt angrenzt und ihn bevorzugt umgibt.

Die lichtabsorbierende Beschichtung kann eine metallische Beschichtung sein. Insbesondere kann sie Chrom enthalten.

Die Umfangsfläche und der daran anschließende Bereich der Innenseite, auf denen die lichtabsorbierende Beschichtung aufgebracht ist, weist eine Mattstruktur (z.B. einen Milchglaseffekt) auf (ist z.B. aufgeraut). Dies kann z.B. dadurch erreicht werden, dass sie geschliffen aber nicht poliert wurden. Zusammen mit der lichtabsorbierenden Beschichtung kann so eine matt-absorbierende Beschichtung bereitgestellt werden. Die lichtabsorbierende Beschichtung ist bevorzugt schwarz. Es kann somit eine matt-schwarze Schicht oder matt-schwarze Oberfläche zur Lichtabsorption bereitgestellt werden.

Bei der erfindungsgemäßen Linse kann der auf der Umfangsfläche ausgebildete Abschnitt der lichtabsorbierenden Beschichtung mit dem auf dem an die Umfangsfläche anschließenden Bereich der Innenseite ausgebildeten Abschnitt der lichtabsorbierenden Beschichtung (z.B. in einer Schnittansicht) einen Winkel einschließen, der im Bereich von 80 bis 100° und bevorzugt im Bereich von 85 bis 95° und besonders bevorzugt im Bereich von 88 bis 92° liegen. Insbesondere kann der Winkel 90°, 91° oder 89° betragen.

Der auf dem der Umfangsfläche anschließenden Bereich der Innenseite ausgebildete Abschnitt der lichtabsorbierenden Beschichtung kann in einer Draufsicht auf die Innenseite gesehen ringförmig sein.

Die Form der lichtabsorbierenden Beschichtung kann als Hohlzylinder mit einem eine Öffnung aufweisenden Boden beschrieben werden. Dabei bildet der Abschnitt der lichtabsorbierenden Beschichtung, der auf der Umfangsfläche ausgebildet ist, die Wandung des Hohlzylinders und bildet der Abschnitt der lichtabsorbierenden Beschichtung, der auf dem der Umfangsfläche anschließenden Bereich der Innenseite ausgebildet ist, den Boden mit der Öffnung. Die Öffnung des Bodens ist natürlich so gelegt, dass die Linse ihre bestimmungsgemäße abbildungsgemäße Eigenschaft realisieren kann. Insbesondere weist die Innenseite einen konkav gekrümmten Abschnitt auf, der nicht mit der lichtabsorbierenden Beschichtung bedeckt ist.

Die lichtabsorbierende Beschichtung kann als einzelne Schicht ausgebildet sein oder als mehrlagige Schicht. In gleicher Weise kann die lötbare Schicht als Einzelschicht oder als mehrlagiges Schichtsystem ausgebildet sein. Bevorzugt weist die äußerste Schicht der lötbaren Schicht Gold auf.

Die erfindungsgemäße Linse (und gegebenenfalls das nachfolgende Optiksystem) ist insbesondere für den sichtbaren Wellenlängenbereich (also Wellenlängen von 400 bis 700 nm) und gegebenenfalls auch noch für den nahen Infrarotbereich (Wellenlängen von 710 bis 3000 nm, 710 bis 900 nm oder 780 bis 900 nm) ausgelegt.

Die erfindungsgemäße Linse kann als Plan-Konkav-Linse ausgebildet sein. Die konkave Krümmung ist bevorzugt eine sphärische Krümmung. Sie kann jedoch auch eine asphärische Krümmung sein. Die konkave Krümmung ist bevorzugt auf der Innenseite der Linse ausgebildet. Die plane Seite ist bevorzugt die Außenseite der Linse. Die Außenseite der Linse kann jedoch auch sphärisch oder asphärisch gekrümmt (z.B. konkav oder konvex) sein.

Die erfindungsgemäße Linse ist bevorzugt einstückig ausgebildet. Sie kann aus Saphir gebildet sein.

Die erfindungsgemäße Linse kann auf ihrer Außenseite und/oder ihrer Innenseite eine Antireflexionsbeschichtung aufweisen. Die Antireflexionsbeschichtung ist bevorzugt nur in Bereichen ausgebildet, in denen keine lichtabsorbierende Beschichtung aufgebracht ist.

Es wird ferner ein Endoskop mit einem Endoskopschaft, der einen ein distales Ende aufweisenden Optikkanal umfasst, bereitgestellt, wobei eine erfindungsgemäße Linse (oder Weiterbildungen der erfindungsgemäßen Linse) mittels der lötbaren Schicht so im distalen Ende eingelötet ist, dass das distale Ende abgedichtet ist. Die Linse kann Teil eines Objektivs sein. Ferner können noch weitere optische Elemente im Optikkanal angeordnet sein, wie z.B. ein Umkehrsystem, das z.B. als Stablinsensystem ausgebildet sein kann.

Die die konkave Krümmung aufweisende Innenseite weist bevorzugt zu den weiteren Elementen des Objektivs oder des Optikkanals. Die plane Außenseite weist bevorzugt zu einem abzubildenden Objekt.

Der Endoskopschaft kann insbesondere als starrer Schaft ausgebildet sein.

Unter einer Abdichtung des distalen Endes wird insbesondere verstanden, dass das distale Ende flüssigkeitsdicht ist und das es autoklavierbar ist.

Es wird ferner eine Endoskopoptik für ein Endoskop bereitgestellt, wobei die Endoskopoptik einen ein distales Ende aufweisenden Optikkanal (bzw. Optikrohr) und eine erfindungsgemäße Linse (oder Weiterbildungen der erfindungsgemäßen Linse) umfasst und wobei die Linse mittels der lötbaren Schicht so im distalen Ende eingelötet ist, dass das distale Ende abgedichtet ist.

Das erfindungsgemäße Endoskop ist bevorzugt ein Geradeausblick-Endoskop oder ein Endoskop mit schräger Blickrichtung. Ferner kann das Endoskop so ausgebildet sein, dass der Winkel der Blickrichtung verstellbar ist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische Schnittansicht eines ersten Ausführungsbeispiels des erfindungsgemäßen Endoskops;
- Fig. 2: eine vergrößerte Schnittansicht des distalen Endes des Endoskopschaftes des Endoskops von Fig. 1;
- Fig. 3: eine vergrößerte Schnittansicht der Linse 7 des Objektivs 6 gemäß Fig. 1 und 2;
- Fig. 4: eine Draufsicht auf die Innenseite der Linse 7, und
- Fig. 5: eine Schnittansicht eines weiteren Ausführungsbeispiels des erfindungsgemäßen Endoskops.

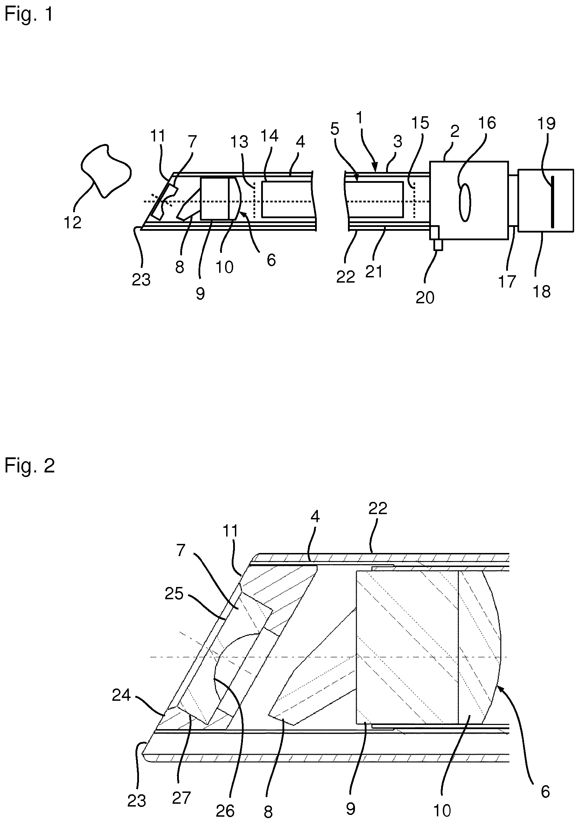

Bei dem in Fig. 1 gezeigten Ausführungsbeispiel umfasst das erfindungsgemäße Endoskop 1, das z.B. als Arthroskop ausgebildet sein kann, ein Hauptteil 2 und einen damit verbundenen Schaft 3.

In dem Schaft 3 erstreckt sich ein Optikrohr 4, in dem eine Endoskopoptik 5 angeordnet ist. Die Endoskopoptik 5 weist ein Objektiv 6 mit einer ersten Linse 7, einem Umlenkprisma 8 sowie einer zweiten und dritten Linse 9, 10 auf, das ein sich vor einem distalen Ende 11 des Optikrohrs 4 befindendes Objekt 12 als distales Zwischenbild in eine distale Zwischenbildebene 13 abbildet. Ferner umfasst die Endoskopoptik 5 ein Umkehrsystem 14, das das distale Zwischenbild aus der distalen Zwischenbildebene 13 als proximales Zwischenbild in eine proximale Zwischenbildebene 15 abbildet. Das Umkehrsystem kann z.B. als Stablinsensystem ausgebildet sein und eine oder mehrere Zwischenabbildungen durchführen, um das gewünschte proximale Zwischenbild zu erzeugen.

Im Hauptteil 2 kann eine weitere Optik 16 (z.B. Okular 16) angeordnet sein. Am vom Schaft 3 wegweisenden Ende des Hauptteils 2 kann ein Kameraanschluss 17 vorgesehen sein, an dem eine Kamera 18 lösbar befestigt ist. Die Kamera 18 kann eine (nicht gezeigte) Optik sowie einen flächigen Bildsensor 19 aufweisen. Der Bildsensor 19 kann beispielsweise ein CCD-Sensor oder ein CMOS-Sensor sein. Die Kamera kann nicht nur, wie in Fig. 1 gezeigt ist, direkt mit dem Kameraanschluss 17 verbunden sein. Es ist auch möglich, dass zwischen Kameraanschluss 17 und der Kamera 18 ein (nicht gezeigter) Koppler zwischengeschaltet ist, der seinerseits eine Optik enthalten kann.

An dem Hauptteil 2 ist ein Beleuchtungsanschluss 20 ausgebildet, der mit Lichtleitfasern 21 (von denen in Fig. 1 stellvertretend nur eine eingezeichnet ist) verbunden ist, die sich vom Beleuchtungsanschluss 20 durch einen Bereich zwischen einem Außenrohr 22 des Schaftes 3 und dem Optikrohr 4 bis zum distalen Ende 23 des Schaftes 3 erstrecken und dort das Licht zur Beleuchtung des Objektes 6 abgeben.

Wie in der vergrößerten Detailansicht des Objektives 6 im distalen Endbereich des Schaftes 3 in Fig. 2 gezeigt ist, weist das distale Ende 11 des Optikrohrs 4 eine Fassung 24 (das z.B. mit dem Optikrohr 4 verschweißt oder verlötet ist) auf, in die die erste Linse 7 eingelötet ist, so dass die erste Linse 7 das distale Ende 11 des Optikrohrs 4 abdichtet. Dadurch kann z.B. keine Feuchtigkeit über das distale Ende 11 in das Optikrohr 4 (das auch als Optikkanal 4 bezeichnet werden kann) eintreten. Insbesondere ist dadurch ein Autoklavieren des Endoskopschaftes 3 möglich.

Die erste Linse 7 weist eine plane oder gekrümmte Außenseite 25, eine Innenseite 26 mit konkaver Krümmung sowie eine die beiden Seiten verbindende Umfangsfläche 27 auf. Die plane Außenseite 25 bzw. die gekrümmte Außenseite 25 sowie die konkav gekrümmte Innenseite 26 sind so ausgebildet, dass die erste Linse 7 eine negative Brennweite aufweist.

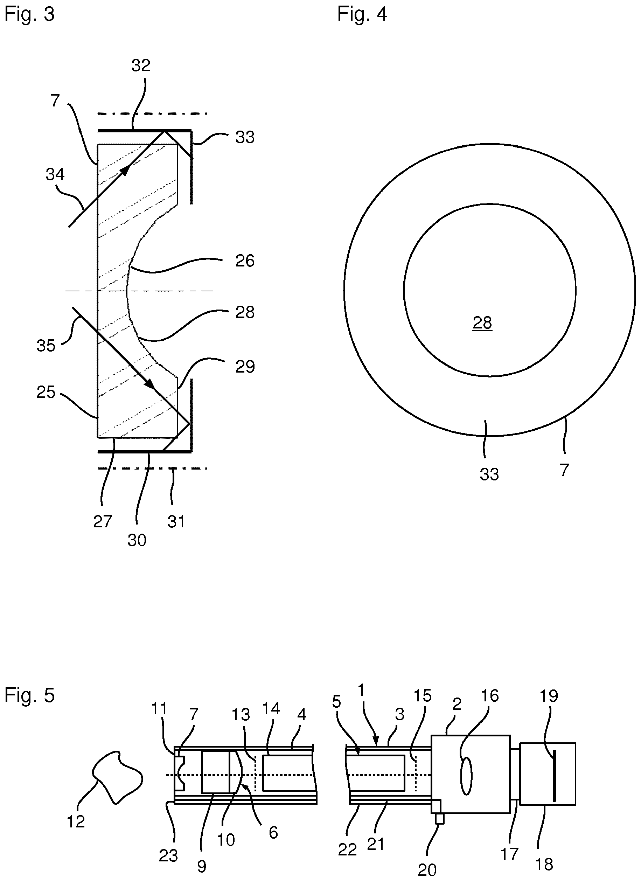

Wie insbesondere der vergrößerten Detailansicht der ersten Linse 7 in Fig. 3 zu entnehmen ist, ist die erste Linse 7 einstückig ausgebildet. Bevorzugt ist sie als Saphir-Linse 7 ausgebildet.

Durch die erste Linse 7 sind große Bildwinkel möglich und es kann auf ein sonst übliches Planglas zur Abdichtung des distalen Endes 11 verzichtet werden, da dieses Abdichten in der beschriebenen Art und Weise durch die in die Fassung 24 eingelötete erste Linse 7 verwirklicht wird. Des Weiteren ist die erste Linse 7 kratzfest, da sie aus Saphir hergestellt ist.

Die Innenseite 26 weist einen in der Mitte liegenden konkav gekrümmten ersten Bereich 28 und einen diesen ersten Bereich 28 ringförmig umgebenden zweiten Bereich 29 auf. Der erste Bereich 28 der Innenseite 26 sowie die Außenseite 25 sind poliert, da sie für die optische Abbildung verwendet werden. Der zweite Bereich 29 hingegen ist nicht poliert, sondern lediglich geschliffen und weist somit eine matte oder eine rau-matte Oberflächenstruktur auf. Man kann auch sagen, dass der zweite Bereich 29 einen Milchglaseffekt aufweist.

Zur Streulichtunterdrückung ist auf der Umfangsfläche 27 sowie auf den daran unmittelbar angrenzenden zweiten Bereich 29 der Innenseite 26 eine lichtabsorbierende Beschichtung 30 aufgebracht. Bei der lichtabsorbierenden Beschichtung 30 kann es sich insbesondere um eine metallische Beschichtung handeln. Die lichtabsorbierende Beschichtung 30 kann eine einzelne Schicht oder ein Schichtaufbau aus mehreren einzelnen Schichten sein. Die lichtabsorbierende Beschichtung 30 kann auch als Schwärzung bezeichnet werden. Beispielsweise kann die lichtabsorbierende Beschichtung 30 als Schwarzchrom-Beschichtung ausgebildet sein.

Da die lichtabsorbierende Beschichtung 30 auf der Umfangsfläche 27 mit der rau-matten Oberflächenstruktur und dem zweiten Bereich 29 mit der rau-matten Oberflächenstruktur aufgebracht ist, liegt eine matt-schwarze Schicht oder matt-schwarze Oberfläche vor, die unerwünschtes Licht ausgezeichnet absorbiert.

Im Bereich der Umfangsfläche 27 ist auf der lichtabsorbierenden Beschichtung 30 eine lötbare Schicht 31 aufgebracht, wobei die lichtabsorbierende Beschichtung 30 (und insbesondere ihr matt-schwarzer Charakter) darunter erhalten bleibt. Die lötbare Schicht 31 kann eine Einzelschicht sein oder kann mehrere Teilschichten aufweisen. Bevorzugt ist die lötbare Schicht, wenn sie eine Einzelschicht ist, als Goldschicht ausgebildet. Wenn die lötbare Schicht 31 mehrere Teilschichten aufweist, ist bevorzugt die äußerste Teilschicht eine Goldschicht. Das Vorsehen der Goldschicht ist für das Löten vorteilhaft, da diese beim Löten nicht oxidiert.

Die lichtabsorbierende Beschichtung 30 und die lötbare Schicht 31 sind in Fig. 3 in Art einer Explosionsdarstellung gezeigt. Natürlich ist die lichtabsorbierende Beschichtung 30 ohne den gezeigten Abstand auf der Umfangsfläche 27 und auf dem zweiten Bereich 29 aufgebracht und ist die lötbare Schicht 31 ohne den gezeigten Abstand auf der lichtabsorbierenden Beschichtung 30 aufgebracht.

Durch den beschriebenen Aufbau der ersten Linse 7 kann die erste Linse 7 in die Fassung 24 gelötet werden, so dass das distale Ende 11 des Optikrohrs 4 hermetisch dicht ausgebildet werden kann.

Wie in der Schnittdarstellung gemäß Fig. 3 sowie in der Draufsicht auf die Innenseite 26 in Fig. 4 gut erkennbar ist, weist die lichtabsorbierende Beschichtung 30 einen ersten Abschnitt 32 (der sich geradlinig in der Richtung von der Innenseite 26 zur Außenseite 25 erstreckt) auf der Umfangsfläche 27 sowie einen zweiten (hier planen) Abschnitt 33 auf dem zweiten Bereich 29 der Innenseite 26 auf, wobei die beiden Abschnitte 32, 33 (in Fig. 3) einen Winkel einschließen, der hier 90° beträgt. Der Winkel kann aber auch im Bereich von 80 bis 100° liegen. Durch das Vorsehen dieser beiden Abschnitte 32 und 33 wird der Vorteil erreicht, dass unerwünschtes Streulicht, das z.B. auf den ersten Abschnitt 32 trifft und von diesem nicht vollständig absorbiert wird, zum zweiten Abschnitt 33 hin reflektiert wird, wie das in Fig. 3 schematisch für einen Streulichtstrahl 34 dargestellt ist. Natürlich kann auch ein Streulichtstrahl 35, der zuerst auf den zweiten Abschnitt 33 trifft und von diesem nicht vollständig absorbiert wird, in vergleichbarer Weise weiter absorbiert werden, da die Reflexion dieses Strahls 35 auf den ersten Abschnitt 32 trifft, der eine weitere Absorption bewirkt. Es wird somit eine sehr gute Streulichtunterdrückung erreicht, so dass die Abbildungseigenschaften der gesamten Endoskopoptik 5 deutlich verbessert sind. Es wird ein höherer Kontrast erreicht und unerwünschte Doppel- oder Geisterbilder können effektiv verhindert werden.

Man kann die Form der lichtabsorbierenden Beschichtung 30 als Hohlzylinder mit einem eine Öffnung aufweisenden Boden beschreiben, wobei der erste Abschnitt 32 die Wandung des Hohlzylinders (z.B. mit kreisförmiger Außenkontur bzw. einem Kreis als Mantellinie) und der zweite Abschnitt 33 den Boden bildet. Da der zweite Abschnitt 33 ringförmig ist (Fig. 4), weist er die Öffnung auf, die so gelegt ist, dass der konkav gekrümmte erste Bereich 28 nicht mit der lichtabsorbierenden Beschichtung 30 beschichtet ist. Die Mantellinie für die Wandung des Hohlzylinders muss nicht kreisförmig sein, sondern kann auch jede andere in sich geschlossene Form aufweisen (z.B. elliptisch, oval oder sogar polygonartig). Der Hohlzylinder kann ein senkrechter Hohlzylinder oder ein schiefer Hohlzylinder sein. Ferner kann statt einem Hohlzylinder auch die Form eines hohlen Kegelstumpfes oder eine sonstige konkave Form mit einem Boden, der die Öffnung aufweist, und eine Mantelfläche realisiert sein.

Die erste Linse 7 kann auf ihrer Außenseite 25 und/oder auf dem ersten Bereich 28 der Innenseite 26 eine Antireflexionsschicht (nicht gezeigt) aufweisen.

Bei dem hier beschriebenen Ausführungsbeispiel liegt ein Teil des zweiten Bereiches 29 der Innenseite 26 an der Fassung 24 an, wie in Fig. 2 gezeigt ist. In diesem Fall kann auf diesen Teil des zweiten Bereiches 29 auch die lötbare Schicht 31 aufgebracht werden, wobei wiederum die lichtabsorbierende Beschichtung 30 darunter erhalten bleibt. Damit kann auch in diesem Bereich eine Lötverbindung mit der Fassung 24 verwirklicht werden.

Das Endoskop 1 kann so abgewandelt werden, dass es keine Fassung 24 aufweist. In diesem Fall wird die erste Linse 7 z.B. direkt mit dem Optikrohr 4 verlötet.

Das bisher beschriebene Ausführungsbeispiel zeigt ein Endoskop 1 mit schräger Blickrichtung gegenüber der Erstreckungsrichtung des Schaftes 3. Natürlich kann das Endoskop 1 auch als Geradeausblick-Endoskop 1 ausgebildet sein, wie schematisch in Fig. 5 gezeigt ist. In diesem Fall ist keine Umlenkung im Objektiv 6 notwendig, so dass das Objektiv 6 hier kein Umlenkprisma 8 aufweist. Ansonsten gleicht der Aufbau dem des bereits beschriebenen Endoskops 1 mit schräger Blickrichtung.

Ferner kann das Endoskop 1 so ausgebildet sein, dass ein optischer Einblick statt der Kamera 18 und dem Kameraanschluss 17 vorgesehen ist. Alternativ kann der Bildsensor 19 in der distalen Zwischenbildebene 13 angeordnet sein. In diesem Fall kann das Umkehrsystem 14 und die weitere Optik 16 weggelassen werden. Die Bilddaten des Bildsensors 19 können z.B. über eine sich durch den Schaft erstreckende Datenverbindung bis zum Hauptteil 2 übertragen werden, dass z.B. eine digitale Anzeige für die Bilddaten aufweist.

## Patentansprüche

1. Linse für ein distales Ende eines Optikkanals eines Endoskopschaftes,
wobei die Linse (7) eine Außenseite (25) und eine Innenseite (26) sowie eine die beiden Seiten (25, 26) verbindende Umfangsfläche (27) aufweist,
wobei auf der Umfangsfläche (27) eine lichtabsorbierende Beschichtung (30) aufgebracht ist,
wobei die Umfangsfläche (27) eine Mattstruktur aufweist,
und wobei auf der lichtabsorbierenden Beschichtung (30) im Bereich der Umfangsfläche (27) eine lötbare Schicht (31) aufgebracht ist, so dass die Linse (7) im distalen Ende (11) so einlötbar ist, dass das distale Ende (11) abgedichtet ist,
**dadurch gekennzeichnet, dass**
der an die Umfangsfläche (27) anschließende Bereich (29) der Innenseite (26) ebenfalls die Mattstruktur aufweist und
dass auf dem an die Umfangsfläche (27) anschließenden Bereich (29) der Innenseite (26) ebenfalls die lichtabsorbierende Beschichtung (30) aufgebracht ist.

2. Linse nach Anspruch 1, bei der die lichtabsorbierende Beschichtung (30) eine metallische Beschichtung ist.

3. Linse nach einem der obigen Ansprüche, bei der die Innenseite (26) einen konkav gekrümmten Abschnitt (28) aufweist.

4. Linse nach einem der obigen Ansprüche, bei der die lötbare Schicht (31) auch auf dem an die Umfangsfläche (27) anschließenden Bereich (29) der Innenseite (26) aufgebracht ist.

5. Linse nach einem der obigen Ansprüche, bei der die lichtabsorbierende Beschichtung Chrom aufweist.

6. Linse nach einem der obigen Ansprüche, bei der der auf der Umfangsfläche (27) ausgebildete Bereich (32) der lichtabsorbierenden Beschichtung (30) mit dem auf dem an die Umfangsfläche anschließenden Bereich (29) der Innenseite (26) ausgebildete Abschnitt (33) der lichtabsorbierenden Beschichtung (30) in einer Schnittansicht einen Winkel einschließt, der im Bereich von 80° bis 100° liegt.

7. Linse nach einem der obigen Ansprüche, bei der der auf dem der Umfangsfläche (27) anschließenden Bereich (29) der Innenseite (26) ausgebildete Abschnitt (13) der lichtabsorbierenden Beschichtung (30) in einer Draufsicht auf die Innenseite (26) gesehen ringförmig ist.

8. Linse nach einem der obigen Ansprüche, bei der die lötbare Schicht (31) Gold aufweist.

9. Endoskop mit
einem Endoskopschaft (3), der einen ein distales Ende (11) aufweisenden Optikkanal (4) umfasst, und
einer Linse (7) nach einem der obigen Ansprüche,
wobei die Linse (7) mittels der lötbaren Schicht (31) so im distalen Ende (11) eingelötet ist, dass das distale Ende (11) abgedichtet ist.

## Claims

1. Lens for a distal end of an optical channel of an endoscope shaft,
wherein the lens (7) comprises an outer side (25) and an inner side (26) as well as a circumferential surface (27) connecting the two sides (25, 26),
wherein a light-absorbing coating (30) is applied to the circumferential surface (27), wherein the circumferential surface (27) has a matt structure,
and wherein a solderable layer (31) is applied to the light-absorbing coating (30) in the area of the circumferential surface (27) with the result that the lens (7) can be soldered in the distal end (11) such that the distal end (11) is sealed,
**characterized in that**
the area (29) of the inner side (26) connected to the circumferential surface (27) also has the matt structure, and
that the light-absorbing coating (30) is also applied to the area (29) of the inner side (26) connected to the circumferential surface (27).

2. Lens according to claim 1, in which the light-absorbing coating (30) is a metallic coating.

3. Lens according to one of the above claims, in which the inner side (26) comprises a concavely curved portion (28).

4. Lens according to one of the above claims, in which the solderable layer (31) is also applied to the area (29) of the inner side (26) connected to the circumferential surface (27).

5. Lens according to one of the above claims, in which the light-absorbing coating comprises chromium.

6. Lens according to one of the above claims, in which the area (32) of the light-absorbing coating (30) formed on the circumferential surface (27) forms, in a sectional view, an angle which lies in the range of from 80° to 100° with the portion (33) of the light-absorbing coating (30) formed on the area (29) of the inner side (26) connected to the circumferential surface.

7. Lens according to one of the above claims, in which the portion (13) of the light-absorbing coating (30) formed on the area (29) of the inner side (26) connected to the circumferential surface (27) is annular, seen in a top view onto the inner side.

8. Lens according to one of the above claims, in which the solderable layer (31) comprises gold.

9. Endoscope with
an endoscope shaft (3), which comprises an optical channel (4) comprising a distal end (11), and
a lens (7) according to one of the above claims,
wherein the lens (7) is soldered in the distal end (11) by means of the solderable layer (31) such that the distal end (11) is sealed.

## Revendications

1. Lentille destinée à une extrémité distale d'un canal optique d'une tige d'endoscope,
la lentille (7) comportant un côté extérieur (25) et un côté intérieur (26) ainsi qu'une surface périphérique (27) reliant les deux côtés (25, 26),
un revêtement photo-absorbant (30) étant appliqué sur la surface périphérique (27),
la surface périphérique (27) présentant une structure mate,
et une couche soudable (31) étant appliquée sur le revêtement photo-absorbant (30) dans la zone de la surface périphérique (27) de sorte que la lentille (7) puisse être soudée dans l'extrémité distale (11) de façon à obturer l'extrémité distale (11) de manière étanche,
**caractérisée en ce que**
la zone (29) du côté intérieure (26) qui est contiguë à la surface périphérique (27) présente également la structure mate et
le revêtement photo-absorbant (30) est également appliqué sur la zone (29) du côté intérieur (26) qui est contiguë à la surface périphérique (27).

2. Lentille selon la revendication 1, dans laquelle le revêtement photo-absorbant (30) est un revêtement métallique.

3. Lentille selon l'une des revendications précédentes, dans laquelle le côté intérieur (26) comporte une portion (28) incurvée de manière concave.

4. Lentille selon l'une des revendications précédentes, dans laquelle la couche soudable (31) est également appliquée sur la région (29) du côté intérieur (26) qui est contiguë à la surface périphérique (27).

5. Lentille selon l'une des revendications précédentes, dans laquelle le revêtement photo-absorbant comprend du chrome.

6. Lentille selon l'une des revendications précédentes, dans laquelle la zone (32) du revêtement photo-absorbant (30) qui est formée sur la surface périphérique (27) forme dans une vue en coupe un angle compris entre 80° et 100° avec la portion (33) du revêtement photo-absorbant (30) qui est formée sur la zone (29) du côté intérieur (26) qui contiguë à la surface périphérique.

7. Lentille selon l'une des revendications précédentes, dans laquelle la portion (13) du revêtement photo-absorbant (30) qui est formée sur la zone (29) du côté intérieur (26) qui est contiguë à la surface périphérique (27) a une forme annulaire dans une vue de dessus du côté intérieur (26).

8. Lentille selon l'une des revendications précédentes, dans laquelle la couche soudable (31) comprend de l'or.

9. Endoscope comprenant
une tige d'endoscope (3) qui comprend un canal optique (4) pourvu d'une extrémité distale (11), et
une lentille (7) selon l'une des revendications précédentes,
la lentille (7) étant soudée dans l'extrémité distale (11) au moyen de la couche soudable (31) de façon à obturer l'extrémité distale (11) de manière étanche.
